# EUROPEAN PATENT SPECIFICATION

(11) **EP 1 252 151 B1**
(45) Date of publication and mention of the grant of the patent: **17.03.2004**
(21) Application number: 01904235.7
(22) Date of filing: 24.01.2001
(51) Int. Cl.: C07D 281/16, C07D 295/10

(54) **A PROCESS FOR THE PREPARATION OF QUETIAPINE AND INTERMEDIATES THEREFOR**
VERFAHREN ZUR HERSTELLUNG VON QUETIAPIN UND INTERMEDIATE DAFÜR
PROCéDé DE PRéPARATION DE QUéTIAPINE ET DES SES INTERMéDIAIRES

(30) Priority: 25.01.2000 HU 0000283
(43) Date of publication of application: 30.10.2002
(73) Proprietor: EGIS GYOGYSZERGYAR RT., 1106 Budapest (HU)
(72) Inventor: BOZSING, Dániel, H-1172 Budapest (HU); KOVANYINE LAX, Györgyi, H-1141 Budapest (HU); SIMIG, Gyula, H-1126 Budapest (HU); RAKOCZY, Györgyné, H-1161 Budapest (HU); TÖMPE, Péter, H-1116 Budapest (HU); KRASZNAI, György, H-1172 Budapest (HU); VERECZKEYNE DONATH, Györgyi, H-1036 Budapest (HU); NAGY, Kálmán, H-1025 Budapest (HU)
(74) Representative: Beszédes, Stephan G., Dr.
(86) International application number: PCT/HU2001/000010
(87) International publication number: WO 2001/055125

(56) References cited:
- CH-A- 422 793
- CH-A- 476 753
- US-A- 3 539 573
- MCEVOY: J. MED. CHEM., vol. 13, 1970, page 295-297 XP000999698
- HIRATA ET AL.: J. PHARM. SCI., vol. 67, 1978, pages 157-162, XP000999669
- ANDERSON ET AL.: ARZNEIM. FORSCH., vol. 12, 1962, pages 937-942, XP000999560
- SCHMUTZ J: "}BER IN 11-STELLUNG AMINO-SUBSTITUIERTE DIBENZO(B,F)-1,4-THIAZAPINEUND -OXAZEPINE" HELVETICA CHIMICA ACTA,CH,VERLAG HELVETICA CHIMICA ACTA. BASEL, vol. 50, no. 1, 2 December 1966 (1966-12-02), pages 245-254, XP000560267 ISSN: 0018-019X
- JILEK ET AL.: COLLECT. CZECH. CHEM. COMMUN., vol. 48, no. 3, 1983, pages 906-927, XP000560351

## Description

### Field of the invention

The invention refers to a novel process for the preparation of 11-[4-/2-(2-hydroxyethoxy)ethyl/-1-piperazinyl]dibenzo[b,f]-1,4-thiazepine of the formula 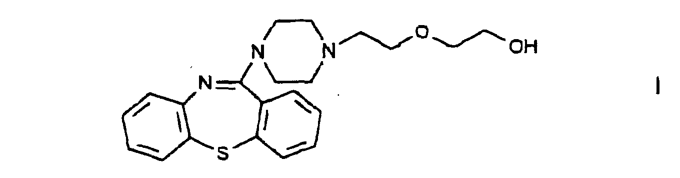 known under the international non-proprietory name quetiapine. The compound has antidopaminerg and/or serotonin receptor antagonist activity, and is used in the clinical practice as an antipsychotic or neuroleptic.

Furthermore, the invention refers to novel intermediates used in the novel process of the invention.

### Background of the invention

According to the process known from EP No. 240 228, the compound of the formula I is prepared by the reaction of the iminochloride of the formula 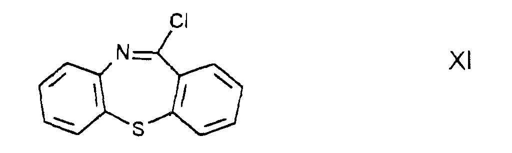 and 1-(2-hydroxyethoxy)ethylpiperazine. The oily crude product that forms is subjected to purification by chromatography using a silica gel column to obtain a yield of 77.7 % on a scale of about 0.5 moles.

The iminochloride of the formula XI used as the starting compound is prepared by the cyclization of the urethane derivative of the formula 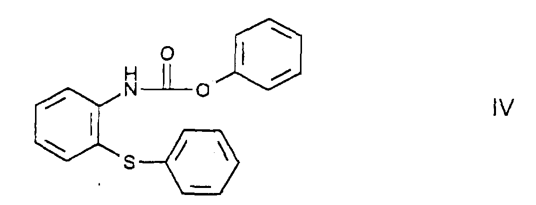 and halogenization of the formed dibenzo[b,f]-1,4-thiazepine-11 (10H)-one of the formula 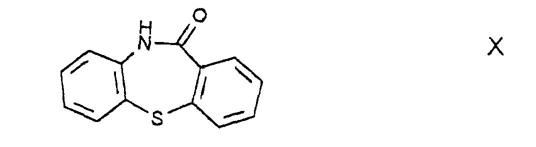 with phosphorus oxychloride according to the data of EP No. 282 236. The yield of the cyclization is 87 %, and that of the halogenization amounts to 92.6 %. Thus, in case of the above known process, the overall yield is 62.6 % calculated for the urethane derivative of the formula IV.

Manufacture on an industrial scale using the known process is rendered difficult and extremely uneconomical, respectively, by the fact that a crystalline product of acceptable purity can be obtained only after purification by column chromatography. The iminochloride of the formula X is rather unstable and hydrolizes by the humidity of the air. When handling larger quantities, this side-reaction reduces the yield and the product of the hydrolysis contaminates the end-product. A further drawback resides in the fact that also the preparation of the 1-(2-hydroxyethoxy)ethylpiperazine can be carried out in several reaction steps which renders the known process still less economical.

According to the other process known from EP No. 282 236, the piperazine derivative of the formula 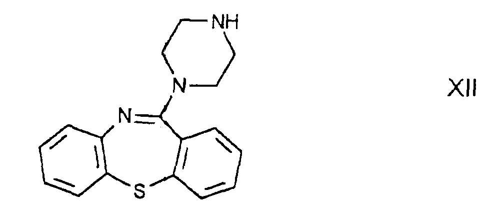 is reacted with 2-haloethoxyethanol, and the product of the formula I is obtained in a yield of 78 %. The piperazine derivative of the formula XII is prepared by reacting the iminochloride of the formula XI with piperazine in a yield of 88 %, thus, the overall yield of the synthesis amounts to merely 55.3% calculated for the urethane derivative of the formula IV.

HIRATA ET AL.: J. PHARM. SCI., vol. 67, 1978, pages 157-162 shows the preparation of the N-{4-[2-(2-hydroxyethoxy)-ethyl]--1-piperazinyl-trimethylen}-2-{trifluormethyl}--phenothiazine [compound III] by treatment of the appropriate chloro compound [compound VI there] with ethylene glycolate {Scheme II}. However, apart form the great structural difference between the compound III there and the thiazepine derivatives of the above formula I according to the respective example a excess of 34 moles of the ethyletic glycol and 5,9 moles of sodium metal calculated on one mole of the 2-chloroethylpiperazinyl compound have been used (page 161, left column).

MCEVOY: J. MED. CHEM., vol. 13, 1970, pages 295-297 discloses processes for the preparation of 11-[4-(2-chloroethyl)-1--piperazinyl]-2-trifluoromethoxydibenz[b,f][1,4]oxazepine (4) (compound IV there) and 11-[4-(2-hydroxyethyl)-1-piperazinyl]--2-trifluoromethoxydibenz[b,f][1,4]oxazepine (3) {compound IV there} and of corresponding aza-derivatives. However, the yield is very low (17% or 33%, respectively) and the course of the reaction is not unambiguous.

From US-A-3 539 573 11-basic substituted dibenzodiazepines the basic substituent of which also can be hydroxyethyl-1--piperazinyl [compound 93] have been known.

The aim of the invention is to provide an economical process for the preparation of quetiapine.

### Summary of the invention

It has been found that the above aim is achieved in the process for the preparation of 11-[4-/2-(2--hydroxyethoxy)ethyl/-1-piperazinyl]dibenzo[b,f]-1,4-thiazepine of the formula I or a pharmaceutically suitable acid addition salt thereof by
a₁) reacting a haloethylpiperazinylthiazepine derivative of the formula 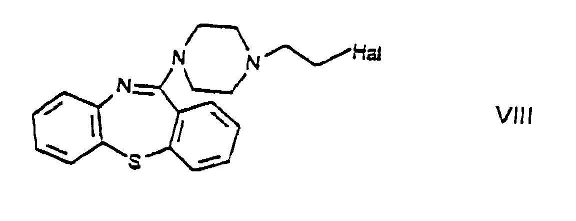 wherein Hal stands for a halo atom, with ethylene glycol in amounts of 20 to 30 moles and sodium in amounts of 1.5 to 1.7 moles, each calculated for 1 mole of haloethylpiperazinylthiazepine derivative of formula VIII; or
a₂) cyclizing a haloethylpiperazine derivative of the formula 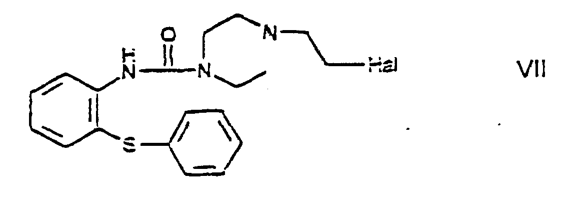 wherein Hal represents a halo atom, in the presence of a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII; or
a₃) reacting the hydroxyethylpiperazine derivative of the formula 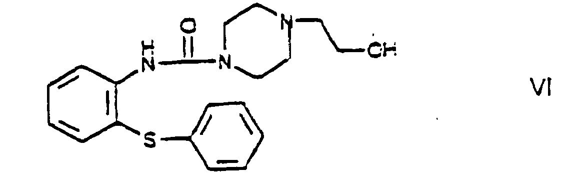 with a halogenating agent, cyclizing the obtained haloethylpiperazine derivative of the formula VII, wherein Hal means a halo atom, in the presence of a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII; or
a₄) reacting the hydroxyethylpiperazine derivative of the formula VI, simultaneously, with a halogenating agent and a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII; or
a₅) reacting the urethane derivative of the formula 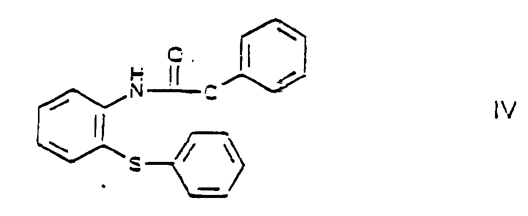 with 1-(2-hydroxyethyl)piperazine of the formula 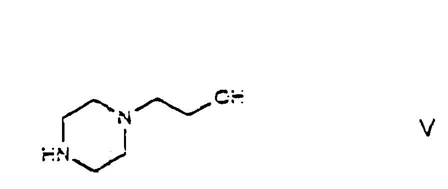 then reacting the formed hydroxyethylpiperazine derivative of the formula VI, simultaneously, with a halogenating agent and a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII;
and, if desired, converting the obtained product to an acid addition salt using a pharmaceutically suitable inorganic or organic acid.

Furthermore, the invention includes the novel piperazine derivatives of the formula 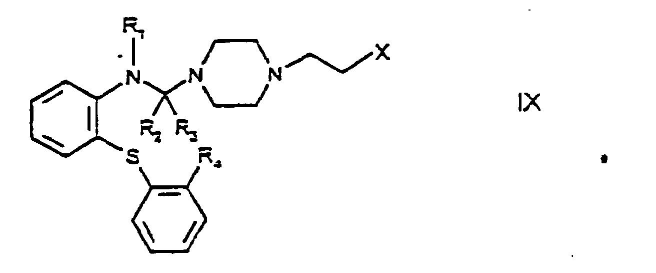 wherein either
R₁ represents a hydrogen atom,
R₂ forms with R₃ an oxygen atom, and
R₄ stands for a hydrogen atom,
X means a hydroxy group or a halo atom; or
R₁ forms with R₂ a valence bond between the adjacent nitrogen and carbon atoms,
R₃ forms with R₄ a valence bond between the adjacent carbon atoms, and
X means a halo atom
and acid addition salts thereof formed with inorganic and organic acids.

The novel piperazine derivatives are intermediates in the novel process of the invention.

### Description of the preferred embodiments

In process a₁) of the invention, the usual reaction terms of Williamson's synthesis are employed. At first, ethylene glycol is converted to alcoholate using sodium metal. Preferably 25 to 27 moles of ethylene glycol are employed. The reaction temperature is mostly 50-150 °C, preferably about 100 °C. As a rule, the reaction proceeds in 5-15 hours, generally in about 9 hours.

In process a₂) of the invention, the preferred starting compound is a haloethylpiperazine derivative of the formula VII, wherein Hal stands for a chloro atom, and the suitable dehydrating agent is phosphorus pentoxide. Suitably, also phosphorus oxychloride is added to the reaction mixture, and the ring closure is carried out preferably at the boiling point of the reaction mixture. The haloethylpiperazinylthiazepine derivative of the formula VIII that forms is converted to the product of the formula I according to the method described in process a₁).

In process a₃) of the invention, suitably thionyl chloride or phosphorus oxychloride, preferably the latter, is used as the halogenating agent. The halogenation reaction is performed in an indifferent organic solvent or an excess of the halogenating agent can be used as the solvent, too. In general, halogenation is carried out at the boiling point of the reaction mixture. The haloethylpiperazine derivative of the formula VII that forms is converted to the product of the formula I according to the method described in process a₂).

In process a₄) of the invention, halogenation of the hydroxyethylpiperazine derivative of the formula VI and subsequent ring closure are carried out in one step without separating the haloethylpiperazine derivative of the formula VII that forms during the halogenation. Suitable halogenating agent is phosphorus oxychloride, preferred dehydrating agent is phosphorus pentoxide. An indifferent organic solvent can be added to the reaction mixture, or an excess of the halogenating agent is used as the solvent. Suitably, the reaction temperature is the boiling point of the reaction mixture. In most cases, the reaction time is 6-10 hours, preferably 7-8 hours. After the end of the reaction, the reaction mixture is poured onto water, made alkaline and extracted with a water-immiscible organic solvent such as dichloromethane. Then, the procedure described in connection with process a₁) of the invention is followed to prepare the product of the formula I.

In process a₅) of the invention, the reaction of the urethane derivative of the formula IV with 1-(2-hydroxyethyl)piperazine is carried out in an indifferent organic solvent, generally an apolar organic solvent, preferably toluene. As a rule, the reaction temperature is higher than room temperature, preferably the boiling point of the solvent employed. The reaction time is relatively short, usually, the reaction proceeds completely in 2 hours. At first, the reaction mixture is washed with aqueous alkali, then with water to remove the phenol formed, the organic phase is dried and evaporated. The residue is crystallized from an organic solvent. The obtained hydroxyethylpiperazine derivative of the formula VI is converted to the product of the formula I by the method described in connection with process a₄) of the invention.

The product of the formula I can be transformed into a pharmaceutically suitable acid addition salt in a manner known *per se.* Preferably, the hemifumarate is prepared. If desired, the base of the formula I can be liberated from the acid addition salt thereof in a manner known *per se*.

The urethane derivative of the formula IV can be prepared by a method known from the literature reacting the 2-amino-diphenyl sulfide of the formula 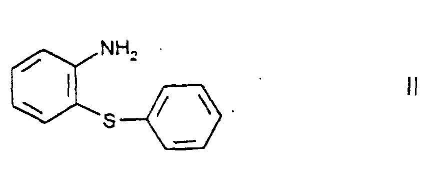 with phenyl chloroformate of the formula

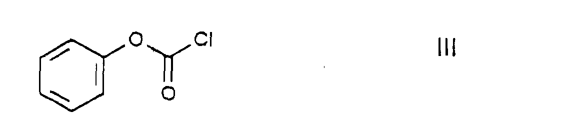

The compound of the formula I is manufactured by the process of the invention in an overall yield of 66-67 % calculated for the urethane derivative of the formula IV. The reaction steps of the process of the invention can be performed easily, the starting compounds and reagents are readily available. The process of the invention does not comprise any procedure that would cause difficulties or would lower the yield. The quetiapine of the formula I that forms is of high purity.

The hydroxyethylpiperazine derivative of the formula VI, the haloethylpiperazine derivatives of the formula VII and the haloethylpiperazinylthiazepine derivatives of the formula VIII - all of which are intermediates in the process of the invention - are novel compounds.

The novel intermediates listed above are characterized by the formula IX. Thus, preferred representatives of the novel piperazine derivatives of the formula IX are the following compounds:
- the hydroxyethylpiperazine derivative of the formula VI and acid addition salts thereof;
- the haloethylpiperazine derivative of the formula VII, wherein Hal is as defined above, and acid addition salts thereof; and
- the haloethylpiperazinylthiazepine derivative of the formula VIII, wherein Hal is as defined above, and acid addition salts thereof.

An especially preferred haloethylpiperazine derivative of the formula VII is N-[4-(2-chloroethyl)piperazine-1-carbonyl]-2-aminodiphenyl sulfide and acid addition salts thereof.

An especially preferred haloethylpiperazinylthiazepine derivative of the formula VIII is 11-[4-(2-chloroethyl)piperazin-1-yl]-dibenzo[b,f]-1,4-thiazepine and acid addition salts thereof.

The novel intermediates can be prepared by the methods described above in connection with the process of the invention.

The invention is further elucidated by means of the following Examples.

### Preparation of the starting compound of the formula IV

### Phenyl 2-phenylthiophenyl carbamate

20.13 g (0.1 moles) of 2-aminodiphenyl sulfide are dissolved in 250 ml of dichloromethane, and the solution formed is cooled to 5 °C. Half of the solution of 18.79 g (15.1 ml, 0.12 moles) of phenyl chloroformate in 26 ml of dichloromethane are added, slowly, to the stirred solution of 2-aminodiphenyl sulfide, then, the other half of the solution of phenyl chloroformate as well as a solution of 3.0 g (0.075 moles) of sodium hydroxide and 9.2 g (0.0875 moles) of sodium carbonate in 50 ml of water are added, simultaneously, taking care that the inner temperature should not exceed 10 °C. After the end of the addition, the reaction mixture is stirred at room temperature for 3 hours, the organic phase is separated, washed three times with diluted hydrochloric acid using a total of 250 ml, dried over anhydrous magnesium sulfate, and evaporated. The residue is crystallized from n-hexane.

Thus, 29 g (90.2 %) of the title compound are obtained. M.p.: 90-91 °C.

| Analysis: for C₁₉H₁₅NO₂S (321.401) | | | | |
|---|---|---|---|---|
| calculated | C 71.01 %, | H 4.70 %, | N 4.36 %, | S 9.98 %; |
| found | C 71.19 %, | H 4.69 %, | N 4.33 %, | S 9.84 %. |

### Example 1

### N-[4-(2-Hydroxyethyl)piperazine-1-carbonyl]-2-aminodiphenyl sulfide - the compound of the formula VI

32.1 g (0.1 moles) of phenyl 2-phenylthiophenyl carbamate are dissolved in 600 ml of toluene, and, to the stirred solution, 13.0 g (0.1 moles) of 1-(2-hydroxyethyl)piperazine are added. The reaction mixture is stirred at boiling temperature for 2 hours, then allowed to cool to room temperature, and washed with 600 ml of 1 N sodium hydroxide solution, then twice with 200 ml of water each time. The organic phase is dried over anhydrous magnesium sulfate, and evaporated. The residue is crystallized from a 10:1 mixture of n-hexane and ethyl acetate, filtered, washed with n-hexane, and dried.

Thus, 33.9 g (94.8 %) of the title compound are obtained in the form of white crystals. M.p.: 96-98 °C.

| Analysis: for C₁₉H₂₃N₃O₂S (357.478) | | | | |
|---|---|---|---|---|
| calculated | C 63.84 %, | H 6.49 %, | N 11.75 %, | S 8.97 %; |
| found | C 63.57 %, | H 6.52 %, | N 11.71 %, | S 9.02 %. |

### Example 2

### N-[4-(2-Chloroethyl)piperazine-1-carbonyl]-2-aminodiphenyl sulfide - a compound of the formula VII

18.8 g (0.05 moles) of N-[4-(2-hydroxyethyl)piperazine-1-carbonyl]-2-aminodiphenyl sulfide are boiled in 65 ml of thionyl chloride for 15 minutes, then evaporated, and the residue is crystallized from n-hexane 18.5 g (89.7 %) of product are obtained which is the hydroch oride of the title compound. M.p.: 180-183°C.

### Formation of base:

To a suspension of 10.31 g (0 025 moles) of the hydrochloride in 250 ml of isopropanol, 2.78 g (0.0275 moles) of triethyl amine are added, the reaction mixture is stirred at room temperature for 1 hour, poured onto water, extracted with dichloromethane, dried over anhydrous magnesium sulfate, and evaporated. Thus, 8.0 g (85.1 %) of the title compound are obtained.

### Formation of the salt with benzenesulfonic acid:

To a solution of 7.5 g (0.02 moles) of the title base in 15 ml of ethanol, a solution of 3.48 g (0.022 moles) of benzenesulfonic acid in 10 ml of ethanol are added. The solution is stirred at room temperature for 1 hour, then cooled with ice water, filtered, and dried. Thus, 6.6 g (60.8 %) of product are obtained which is the benzenesulfonate of the title compound. M.p.: 110-112°C.

| Analysis: for C₂₅H₂₈ClN₃O₄S₂ (534.101) | | | | | |
|---|---|---|---|---|---|
| calculated | C 56.22%, | H 5.28%, | N 7.87%, | Cl 6.64%, | S 12.01%; |
| found | C 55.96%, | H 5.35%, | N 7.73%, | Cl 6.50%, | S 12.05%. |

### Example 3

### 11-[4-(2-Chloroethyl)-1-piperazinyl]-dibenzo[b,f]-1,4-thiazepine - a compound of the formula VIII

### Method A)

A mixture of 8.2 g (0.02 moles) of N-[4-(2-chloroethyl)-piperazine-1-carbonyl]-2-aminodiphenyl sulfide hydrochloride, 84 ml of phosphorus oxychloride and 8.5 g (0.06 moles) of phosphorus pentoxide is reacted at boiling temperature for 15 hours. The solution is allowed to cool, then thoroughly evaporated, the residue is poured onto ice water, the solution is made alkaline by the addition of aqueous ammonia, and extracted with dichloromethane. The organic phase is evaporated, the residue is crystallized from diisopropyl ether, filtered, and dried.

Thus, 5.4 g (75.4 %) of the title compound are obtained. M.p.: 113-115 °C.

### Method B)

A mixture of 35.7 g (0.1 moles) of N-[4-(2-hydroxyethyl)-piperazine-1-carbonyl]-2-aminodiphenyl sulfide, 200 ml of phosphorus oxychloride and 31.2 g (0.22 moles) of phosphorus pentoxide is boiled for 7 hours. The solution is allowed to cool, evaporated, the residue is treated with ice water, made alkaline with aqueous ammonia, extracted with dichloromethane, dried over anhydrousimagnesium sulfate, and evaporated again.The residue is crystallized from diisopropyl ether, the crystals are filtered and dried.

Thus, 28.6 g (80 %) of the title compound are obtained. M.p.: 114-116 °C.

| Analysis: for C₁₉H₂₀ClN₃S (357.908) | | | | | |
|---|---|---|---|---|---|
| calculated | C 63.76%, | H 5.63% | N 11.74%, | Cl 9.91%, | S 8.96%; |
| found | C 63.70%, | H 5.67%, | N 11.68%, | Cl 9.89%, | S 9.07%. |

### Example 4

### 11-[4-/2-(2-Hydroxyethoxy)ethyl/-1-piperazinyl]dibenzo[b,f]-1,4-thiazepine hemifumarate - the compound of the formula I

1.17 g of sodium metal are dissolved in 50 ml of ethylene glycol, and, to the solution obtained, a solution of 10.7 g (0.03 moles) of 11-[4-(2-chloroethyl)-1-piperazinyl]-dibenzo[b,f]-1,4-thiazepine in 60 ml of toluene is added. The reaction mixture is stirred at 100 °C for 9 hours, then, after cooling, 210 ml of water are added. After separation, the toluene phase is extracted with diluted hydrochloric acid, the solution is made alkaline by the addition of aqueous ammonia, extracted with dichloromethane, the organic solution is dried over anhydrous magnesium sulfate, and evaporated under reduced pressure.

Thus, 11.27 g (98 %) of the title base are obtained.

### Formation of salt:

10 g (0.026 moles) of the base obtained are dissolved in 130 ml of ethanol, and, to the solution, 3.13 g (0.027 moles) of fumaric acid are added. The mixture is stirred at boiling point for 25 minutes, then allowed to cool to room temperature. The mixture is maintained in a refrigerator for a night, then the crystals are filtered, washed with cold ethanol, and dried.

Thus, 9.8 g (85.4 %) of the title compound are obtained. M.p.: 172-174 °C.

| Analysis: for C₄₆H₅₄N₆O₈S₂ (883.107) | | | | |
|---|---|---|---|---|
| calculated | C 62.56 %, | H 6.16 %, | N 9.52 %, | S 7.26 %; |
| found | C 62.19 %, | H 6.19 %, | N 9.57 %, | S 7.24 %. |

## Claims

1. A process for the preparation of 11-[4-/2-(2--hydroxyethoxy)-ethyl/-1-piperazinyl]dibenzo[b,f]-1,4-thiazepine of the formula 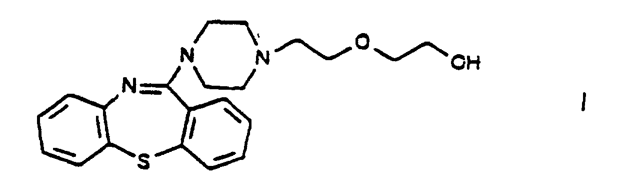 or a pharmaceutically suitable acid addition salt thereof, **characterized by**
a₁) reacting a haloethylpiperazinylthiazepine derivative of the formula 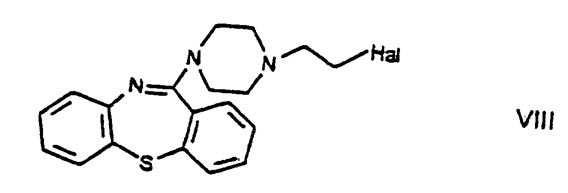 wherein Hal stands for a halo atom, with ethylene glycol in amounts of 20 to 30 moles and sodium in amounts of 1.5 to 1.7 moles, each calculated for 1 mole of haloethylpiperazinylthiazepine derivative of formula VIII; or
a₂) cyclizing a haloethylpiperazine derivative of the formula 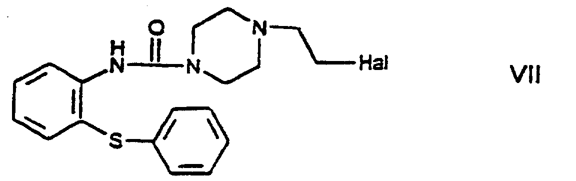 wherein Hal represents a halo atom, in the presence of a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII; or
a₃) reacting the hydroxyethylpiperazine derivative of the formula 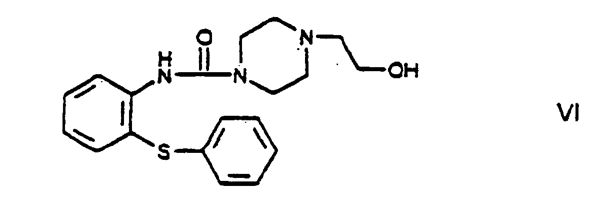 with a halogenating agent, cyclizing the obtained haloethylpiperazine derivative of the formula VII, wherein Hal means a halo atom, in the presence of a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII; or
a₄) reacting the hydroxyethylpiperazine derivative of the formula VI, simultaneously, with a halogenating agent and a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII; or
as) reacting the urethane derivative of the formula 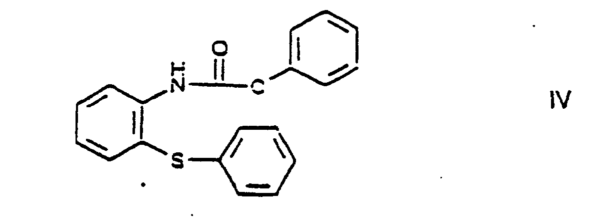 with 1-(2-hydroxyethyl)piperazine of the formula 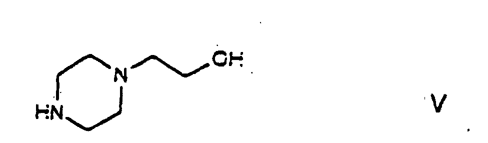 then reacting the formed hydroxyethylpiperazine derivative of the formula VI, simultaneously, with a halogenating agent and a dehydrating agent, and reacting the obtained haloethylpiperazinylthiazepine derivative of the formula VIII, wherein Hal is as defined above, with ethylene glycol in amounts of 20 to 30 moles and sodium in amounts of 1.5 to 1.7 moles, each calculated for 1 mole of haloethylpiperazinylthiazepine derivative of formula VIII;
and, if desired, converting the obtained product to an acid addition salt using a pharmaceutically suitable inorganic or organic acid.

2. A process as claimed in Claim 1 a₂) in which the starting compound is a haloalkylpiperazine derivative of the formula VII, wherein Hal stands for a chloro atom, and the dehydrating agent is phosphorus pentoxide.

3. A process as claimed in Claim a₃) in which the halogenating agent is phosphorus oxychloride.

4. A piperazine derivative of the formula 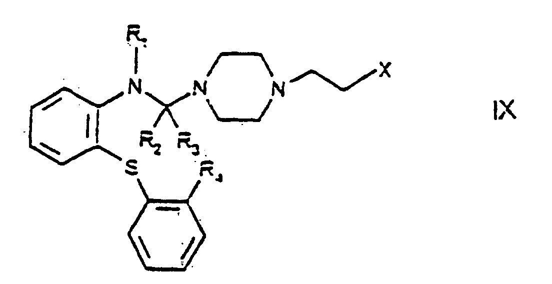 wherein either
R₁ represents a hydrogen atom,
R₂ forms with R₃ an oxygen atom, and
R₄ stands for a hydrogen atom,
X means a hydroxy group or a halo atom; or
R₁ forms with R₂ a valence bond between the adjacent nitrogen and carbon atoms,
R₃ forms with R₄ a valence bond between the adjacent carbon atoms, and
X means a halo atom
and acid addition salts thereof formed with inorganic and organic acids.

5. A hydroxyethylpiperazine derivative of the formula VI as claimed in Claim 4 and acid addition salts thereof.

6. A haloethylpiperazine derivative of the formula VII, wherein Hal stands for a halo atom, as claimed in Claim 4 and acid addition salts thereof.

7. A haloethylpiperazinylthiazepine derivative, wherein Hal stands for a halo atom, as claimed in Claim 4 and acid addition salts thereof.

8. N-[4-(2-Chloroethyl)piperazin-1-cerbonyl]-2-aminodiphenyl sulfide as claimed in Claim 6 and acid addition salts thereof.

9. 11-[4-(2-Chloroethyl)-1-piperazinyl]dibenzo[b,f]-1,4--thiazepine as claimed in Claim 7 and acid addition salts thereof.

## Patentansprüche

1. Verfahren zur Herstellung von 11-[4-/2-(2-Hydroxyethoxy)-ethyl/-1-piperazinyl]dibenzo[b,f]-1,4-thiazepin der Formel 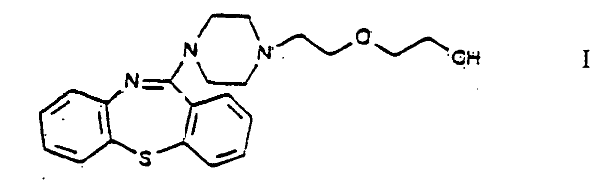 oder eines pharmazeutisch geeigneten Säureadditionssalzes davon, **gekennzeichnet durch**
a₁) Umsetzen eines Halogenethylpiperazinylthiazepin-Derivats der Formel 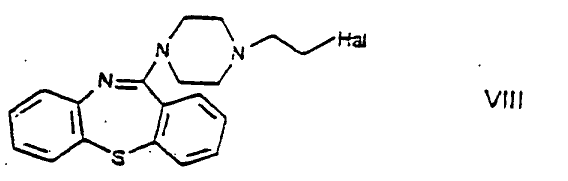 worin Hal für eine Halogenatom steht, mit Ethylenglykol in Mengen von 20 bis 30 Mol und Natrium in Mengen von 1,5 bis 1,7 Mol, wobei jedes für 1 Mol Halogenethylpiperazinylthiazepin-Derivat der Formel VIII berechnet wurde; oder
a₂) Cyclisieren eines Halogenethylpiperazin-Derivats der Formel 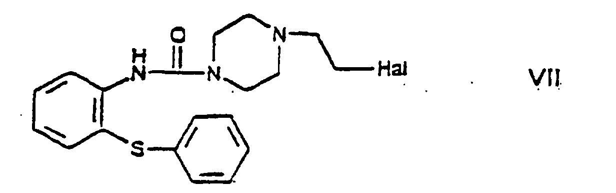 worin Hal für ein Halogenatom steht, in Gegenwart eines Dehydratisierungsmittels, und Umsetzen des erhaltenen Halogenethylpiperazinylthiazepin-Derivats der Formel VIII. worin Hal wie oben definiert ist, mit Ethylenglykol in Mengen von 20 bis 30 Mol und Natrium in Mengen von 1,5 bis 1,7 Mol, wobei jedes für ein Mol Halogenethylpiperazinylthiazepin-Derivat der Formel VIII berechnet ist; oder
a₃) Umsetzen des Hydroxyethylpiperazin-Derivats der Formel 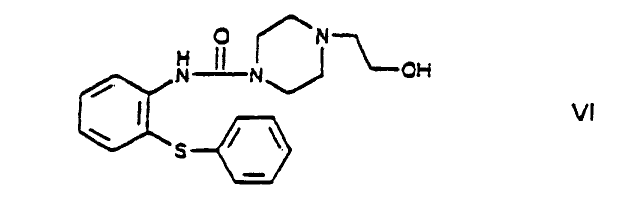 mit einem Halogenierungsmittel, Cyclisieren des erhaltenen Halogenethylpiperazin-Derivats der Formel VII, worin Hal für ein Halogenatom steht, in Gegenwart eines Dehydratisierungsmittels, und Umsetzen des erhaltenen Halogenethylpiperazinylthiazepin-Derivats der Formel VIII, worin Hal wie oben definiert ist, mit Ethylenglykol in Mengen von 20 bis 30 Mol und Natrium in Mengen von 1,5 bis 1,7 Mol, jeweils berechnet für 1 Mol Halogenethylpiperazinylthiazepin-Derivat der Formel VIII; oder
a₄) Umsetzen des Hydroxyethylpiperazin-Derivats der Formel VI gleichzeitig mit einem Halogenisierungsmittel und einem Dehydratisierungsmittel, und Umsetzen des erhaltenen Halogenethylpiperazinylthiazepin-Derivats der Formel VIII, wobei Hal wie oben definiert ist, mit Ethylenglykol in Mengen von 20 bis 30 Mol und Natrium in Mengen von 1,5 bis 1,7 Mol, jeweils berechnet für 1 Mol Halogenethylpiperazinylthiazepin-Derivat der Formel VIII; oder
a₅) Umsetzen des Urethan-Derivats der Formel 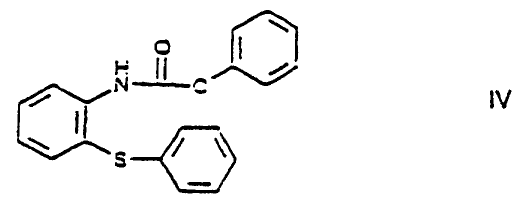 mit 1-(2-Hydroxyethyl)piperazin der Formel 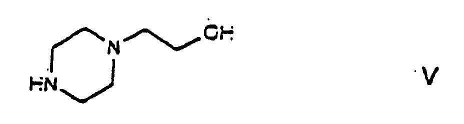 anschließend Umsetzen des gebildeten Hydroxyethylpiperazin-Derivats der Formel VI gleichzeitig mit einem Halogenierungsmittel und einem Dehydratisierungsmittel, und Umsetzen des erhaltenen Halogenethylpiperazinylthiazepin-Derivats der Formel VIII, wobei Hal wie oben definiert ist, mit Ethylenglykol in Mengen von 20 bis 30 Mol und Natrium in Mengen von 1,5 bis 1,7 Mol, jeweils berechnet für 1 Mol Halogenethylpiperazinylthiazepin-Derivat der Formel VIII; und, sofern erwünscht, Umwandeln des erhaltenen Produktes zu einem Säuresadditionssalz unter Verwendung einer pharmazeutisch geeigneten anorganischen oder organischen Säure.

2. Verfahren, wie in Anspruch 1a₂) beansprucht, in dem die Ausgangsverbindung ein Halogenalkylpiperazin-Derivat der Formel VII ist, wobei Hal für ein Chloratom steht und das Dehydratisierungsmittel Phosphorpentoxid ist.

3. Verfahren, wie in Anspruch a₃) beansprucht, in dem das Halogenisierungsmittel Phosphoroxychlorid ist.

4. Piperazinderivat der Formel 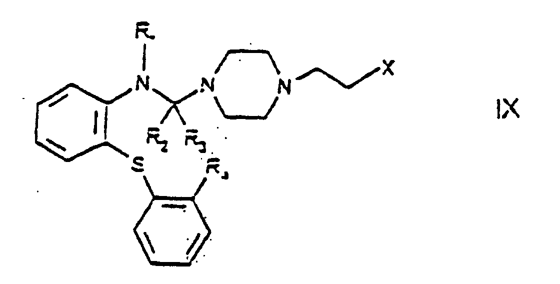 worin entweder
R₁ für ein Wasserstoffatom steht,
R₂ mit R₃ ein Sauerstoffatom bildet, und
R₄ für ein Wasserstoffatom steht,
X für eine Hydroxygruppe oder ein Halogenatom steht; oder
R₁ mit R₂ eine Wertigkeitsbindung zwischen den benachbarten Stickstoff- und Kohlenstoffatomen bildet,
R₃ mit R₄ eine Wertigkeitsbindung zwischen den benachbarten Kohlenstoffatomen bildet und
X für ein Halogenatom steht,
und Säureadditionssalze davon, gebildet mit anorganischen und organischen Säuren.

5. Hydroxyethylpiperazin-Derivat der Formel VI, wie in Anspruch 4 beansprucht, und Säureadditionssalze davon.

6. Halogenethylpiperazin-Derivat der Formel VII, worin Hal für ein Halogenatom steht, wie in Anspruch 4 beansprucht, und Säureadditionssalze davon.

7. Halogenethylpiperazinylthiazepin-Derivat, worin Hal für ein Halogenatom steht, wie in Anspruch 4 beansprucht, und Säureadditionssalze davon.

8. N-[4-(2-Chlorethyl)piperazin-1-cerbonyl]-2-aminodiphenylsulfid, wie in Anspruch 6 beansprucht, und Säureadditionssalze davon.

9. 11-[4-(2-Chlorethyl)-1-piperazinyl]dibenzo[b,f]-1,4-thiazepin, wie in Anspruch 7 beansprucht, und Säureadditionssalze davon.

## Revendications

1. Procédé pour la préparation de la 11-[4-/2-(2-hydroxyéthoxy)-éthyl/-1-pipérazinyl]dibenzo[b,f]-1,4-thiazépine de formule : 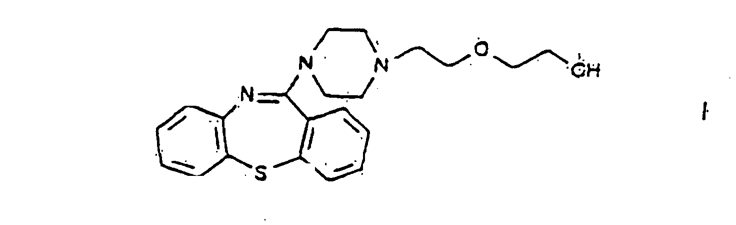 ou d'un sel d'addition acide de celle-ci convenable du point de vue pharmaceutique,
**caractérisé par** :
a₁) la réaction d'un dérivé d'haloéthylpipérazinylthiazépine de formule : 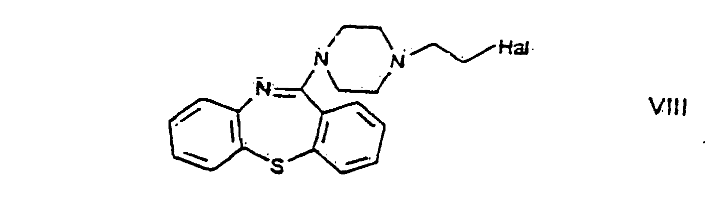 dans laquelle Hal représente un atome d'halogène, avec de l'éthylène glycol en des quantités de 20 à 30 moles et du sodium en des quantités de 1,5 à 1,7 moles, chacune étant calculée pour 1 mole de dérivé d'haloéthylpipérazinylthiazépine de formule VIII ; ou
a₂) la cyclisation d'un dérivé d'haloéthylpipérazine de formule : 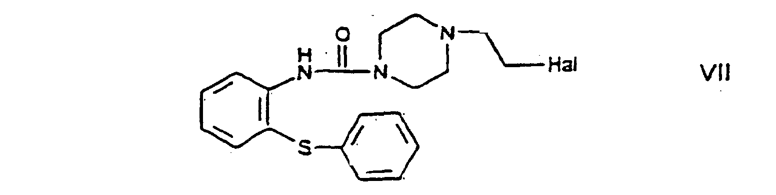 dans laquelle Hal représente un atome d'halogène, en présence d'un agent de déshydratation, et la réaction du dérivé d'haloéthylpipérazinylthiazépine obtenu de formule VIII, dans laquelle Hal est tel que défini plus haut, avec de l'éthylène glycol en des quantités de 20 à 30 moles et du sodium en des quantités de 1,5 à 1,7 moles, chacune étant calculée pour 1 mole de dérivé d'haloéthylpipérazinylthiazépine de formule VIII ; ou
a₃) la réaction du dérivé d'hydroxyéthylpipérazine de formule : 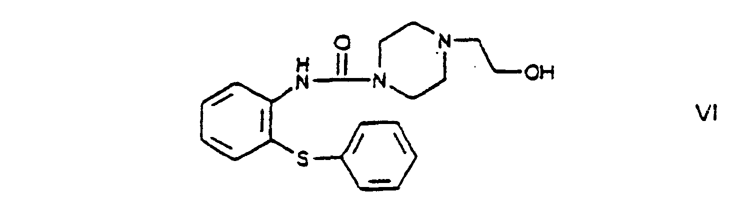 avec un agent d'halogénation, la cyclisation du dérivé d'haloéthylpipérazine obtenu de formule VII, dans laquelle Hal représente un atome d'halogène, en présence d'un agent de déshydratation, et la réaction du dérivé d'haloéthylpipérazinylthiazépine obtenu de formule VIII, dans laquelle Hal est tel que défini plus haut, avec de l'éthylène glycol en des quantités de 20 à 30 moles et du sodium en des quantités de 1,5 à 1,7 moles, chacune étant calculée pour 1 mole de dérivé d'haloéthylpipérazinylthiazépine de formule VIII ; ou
a₄) la réaction du dérivé d'hydroxyéthylpipérazine de formule VI, simultanément, avec un agent d'halogénation et un agent de déshydratation, et la réaction du dérivé d'haloéthylpipérazinylthiazépine obtenu de formule VIII, dans laquelle Hal est tel que défini plus haut, avec de l'éthylène glycol en des quantités de 20 à 30 moles et du sodium en des quantités de 1,5 à 1,7 moles, chacune étant calculée pour 1 mole de dérivé d'haloéthylpipérazinylthiazépine de formule VIII ; ou
a₅) la réaction du dérivé d'uréthane de formule : 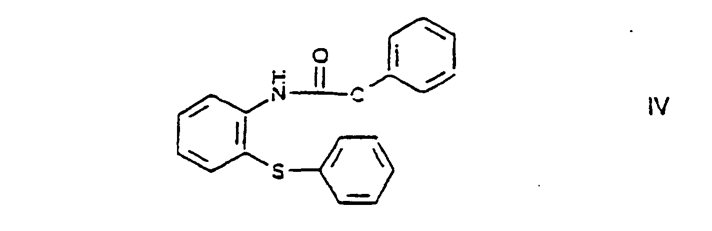 avec la 1-(2-hydroxyéthyl)-pipérazine de formule : 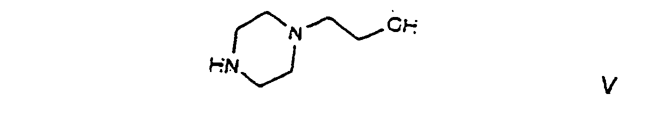 puis la réaction du dérivé d'hydroxyéthylpipérazine formé de formule VI, simultanément, avec un agent d'halogénation et un agent de déshydratation, et la réaction du dérivé d'haloéthylpipérazinylthiazépine obtenu de formule VIII, dans laquelle Hal est tel que défini plus haut, avec de l'éthylène glycol en des quantités de 20 à 30 moles et du sodium en des quantités de 1,5 à 1,7 moles, chacune étant calculée pour 1 mole de dérivé d'haloéthylpipérazinylthiazépine de formule VIII ;
et ; si cela est souhaité, la conversion du produit obtenu en un sel d'addition acide formé avec un acide inorganique ou organique convenable du point de vue pharmaceutique.

2. Procédé selon la revendication 1 a₂) dans lequel le composé de départ est un dérivé d'haloalkylpipérazine de formule VII, dans laquelle Hal représente un atome de chlore, et l'agent de déshydratation est le pentoxyde de phosphore.

3. Procédé selon la revendication 1 a₃) dans lequel l'agent d'halogénation est l'oxychlorure de phosphore.

4. Dérivé de pipérazine de formule : 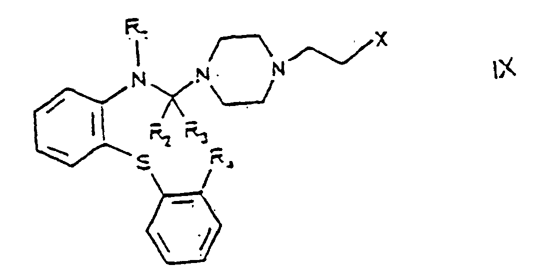 dans laquelle, soit
R₁ représente un atome d'hydrogène,
R₂ forme avec R₃ un atome d'oxygène, et
R₄ représente un atome d'hydrogène,
X signifie un groupe hydroxy ou un atome d'halogène,
soit
R₁ forme avec R₂ une liaison de valence entre les atomes adjacents d'azote et de carbone,
R₃ forme avec R₄ une liaison de valence entre les atomes adjacents de carbone, et
X signifie un atome d'halogène,
et sels d'addition acide de celui-ci formés avec des acides inorganiques et organiques.

5. Dérivé d'hydroxyéthylpipérazine de formule VI selon la revendication 4, et sels d'addition acide de celui-ci.

6. Dérivé d'haloéthylpipérazine de formule VII, dans laquelle Hal représente un atome d'halogène, selon la revendication 4, et sels d'addition acide de celui-ci.

7. Dérivé d'haloéthylpipérazinylthiazépine dans lequel Hal représente un atome d'halogène, selon la revendication 4, et sels d'addition acide de celui-ci.

8. Sulfure de N-[4-(2-chloroéthyl)pipérazin-1-carbonyl]-2-aminodiphényle selon la revendication 6 et sels d'addition acide de celui-ci.

9. 11-[4-(2-Chloroéthyl)-1-pipérazinyl]dibenzo[b,f]-1,4-thiazépine selon la revendication 7 et sels d'addition acide de celle-ci.
